# EUROPEAN PATENT APPLICATION

(11) **EP 4 000 516 A1**
(43) Date of publication of application: **25.05.2022**
(21) Application number: 21788021.0
(22) Date of filing: 08.04.2021
(51) Int. Cl.: A61B 5/145

(54) **BIO-INFORMATION MEASUREMENT SYSTEM AND BIO-INFORMATION CORRECTION METHOD**

(30) Priority: 16.04.2020 CN 202010297985
(71) Applicant: Seknova Biotechnology Co., Ltd., Taipei City, Taiwan 105609 (TW)
(72) Inventor: HUANG, Ching-Chun, Hsinchu, Taiwan 30077 (CN); WANG, Ping-Chun, Hsinchu, Taiwan 30077 (CN); HUANG, Yu-Ching, Hsinchu, Taiwan 30077 (CN)
(74) Representative: Reichert & Lindner Partnerschaft Patentanwälte
(86) International application number: PCT/CN2021/085886
(87) International publication number: WO 2021/208790

(57) **Abstract**

A bioinformatics measurement device comprises at least one measurement device and a host. The measurement device is used to measure bioinformatics of an individual subject. The host receives bioinformatics measured by the measurement devices through wireless communication links established between the host and the measurement devices. The host is selectively switched to a pairing mode or a beacon mode to establish the wireless communication links with the measurement devices according to the number of links between the host and the measurement devices, whereby the bioinformatics measurement device is adapted to different application scenarios. A bioinformatics calibration method is also disclosed, which predicts future bioinformatics according to the slope changes of the bioinformatics, whereby the predicted bioinformatics can approach the current bioinformatics.

## Description

### BACKGROUND OF THE INVENTION

### 1. FIELD OF THE INVENTION

The present invention relates to a measurement system and a calibration method, particularly to a bioinformatics measurement system and a bioinformatics calibration method.

### 2. DESCRIPTION OF THE PRIOR ART

In the conventional method of acquiring bioinformatics (such as blood glucose), the medical personnel uses a syringe to suck blood of the testee or the testee himself punctures his skin to obtain a small amount of blood for chemical analysis. The conventional method can only acquire the bioinformatics occurring at a single time point. If the tests are undertaken very frequently in a short interval of time, the tester should feel inconvenient, and the testee would suffer more pain. Thus, the testees are likely to refuse too frequent tests. Then, the number of tests will be reduced.

So far, bioinformatics sensor patches have been developed to overcome the abovementioned problem. The bioinformatics sensor patch is attached to the skin of an organism and uses low-invasiveness punctures to reduce pain of the testee and acquire the tissue fluid of the testee. Thus, the bioinformatics sensor patch can obtain the bioinformatics of the testee continuously in a longer interval of time. In general, the bioinformatics sensor patch has to transmit bioinformatics to a host, whereby the user can read the measurement results. In different application scenarios, such as usages by individuals or nursing organizations, the bioinformatics sensor patch may be linked to the host in specific ways, which may lead to inconvenience and limitation in application.

In addition to the instant bioinformatics, such as electrocardiogram (ECG) and electroencephalogram (EEG), the bioinformatics sensor patch may also be used to measure lagging bioinformatics, such as measuring blood glucose from sweat or tissue fluid. For a bioinformatics sensor patch, there is a lag between acquisition and occurrence of bioinformatics. Therefore, how to make the bioinformatics acquired by a bioinformatics sensor patch approach the current vital sign has become an important topic in the concerned fields.

Accordingly, developing a bioinformatics sensor patch able to overcome the abovementioned problems has become a target the manufacturers is eager to achieve.

### SUMMARY OF THE INVENTION

The present invention provides a bioinformatics measurement system, which may switch to a pairing mode or a beacon mode according to the number of the links of the measurement system, whereby the bioinformatics measurement system of the present invention can be adapted to different application scenarios.

The present invention provides a bioinformatics calibration method, which predicts a prediction slope of the bioinformatics of the next time interval according to the slope changes of the bioinformatics of the preceding time intervals, and which uses the prediction slope to work out the bioinformatics of the next time interval, whereby the bioinformatics of the next time interval can approach the current bioinformatics.

In one embodiment, the bioinformatics measurement system of the present invention comprises at least one measurement device and a host. The measurement device is used to measure the bioinformatics of an individual subject. Wireless communication links are established between the host and the measurement devices, whereby the host can receive the bioinformatics measured by the measurement devices. The host is selectively switched to a pairing mode or a beacon mode to establish wireless communication links with at least one measurement device according to the number of links with the measurement devices.

In one embodiment, the bioinformatics calibration method of the present invention comprises steps: acquiring a plurality of pieces of bioinformatics of an individual subject respectively in a preceding time interval and a current time interval by an electronic device, wherein the bioinformatics is lagging bioinformatics; working out the slopes of bioinformatics of the preceding time interval and the current time interval by the electronic device according to the plurality of pieces of bioinformatics; predicting a prediction slope of the bioinformatics of a next time interval by the electronic device according to a change of the slopes of the preceding time interval and the current time interval; and working out the bioinformatics behind the next time interval by the electronic device according to the prediction slope.

The objective, technologies, features and advantages of the present invention will become apparent from the following description in conjunction with the accompanying drawings wherein certain embodiments of the present invention are set forth by way of illustration and example.

### BRIEF DESCRIPTION OF THE DRAWINGS

The foregoing conceptions and their accompanying advantages of this invention will become more readily appreciated after being better understood by referring to the following detailed description, in conjunction with the accompanying drawings, wherein:
Fig. 1 is a diagram schematically showing a bioinformatics measurement system according to one embodiment of the present invention;
Fig. 2 schematically shows communication timing diagrams of a bioinformatics measurement system in a beacon mode according to one embodiment of the present invention;
Fig. 3 schematically shows steps of an encrypted linking process of a bioinformatics measurement system according to one embodiment of the present invention;
Fig. 4 is a flowchart of a bioinformatics calibration method according to one embodiment of the present invention;
Fig. 5 is a diagram schematically showing variation of the bioinformatics measured by the bioinformatics measurement device;
Fig. 6 is a diagram schematically showing a bioinformatics measurement system according to another embodiment of the present invention;
Fig. 7 is a flowchart of a bioinformatics calibration method performed on a server side according to one embodiment of the present invention; and
Fig. 8 is a flowchart of a bioinformatics calibration method performed on a host side according to one embodiment of the present invention.

### DESCRIPTION OF THE PREFERRED EMBODIMENTS

Various embodiments of the present invention will be described in detail below and illustrated in conjunction with the accompanying drawings. In addition to these detailed descriptions, the present invention can be widely implemented in other embodiments, and apparent alternations, modifications and equivalent changes of any mentioned embodiments are all included within the scope of the present invention and based on the scope of the Claims. In the descriptions of the specification, in order to make readers have a more complete understanding about the present invention, many specific details are provided; however, the present invention may be implemented without parts of or all the specific details. In addition, the well-known steps or elements are not described in detail, in order to avoid unnecessary limitations to the present invention. Same or similar elements in Figures will be indicated by same or similar reference numbers. It is noted that the Figures are schematic and may not represent the actual size or number of the elements. For clearness of the Figures, some details may not be fully depicted.

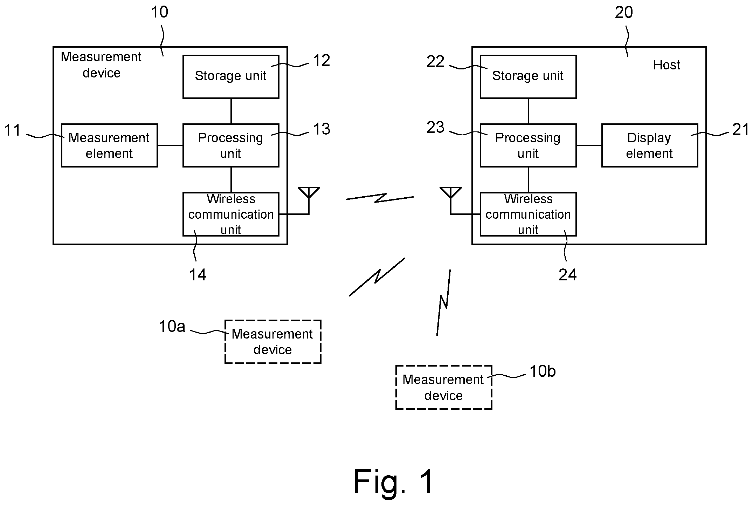

Refer to Fig. 1. In one embodiment, the bioinformatics measurement system of the present invention comprises at least one measurement device 10 and a host 20. The measurement device 10 is used to measure the bioinformatics of an individual subject, which may be at least one of blood glucose and blood ketone but is not limited to them. For example, the measurement device 10 may be a bioinformatics sensor patch or other implantable bioinformatics sensing elements. Wireless communication links are established between the host 20 and the measurement device 10, whereby the host 20 can receive the bioinformatics measured by the measurement device 10.

The measurement device 10 comprises a measurement element 11, a storage unit 12, a processing unit 13 and a wireless communication unit 14. The measurement element 11 is designed according to the measurement target. For example, the measurement element 11 may be a microprobe coated with an appropriate reagent. The processing unit 13 processes the bioinformatics measured by the measurement element 11 to store the bioinformatics in the storage unit 12 and/or transmit the bioinformatics to the host 20.

The host 20 comprises a display element 21, a storage unit 22, a processing unit 23 and a wireless communication unit 24. The host 20 receives the bioinformatics measured by the measurement device 10 through the wireless communication unit 24. The processing unit 23 processes the bioinformatics, then stores the bioinformatics in the storage unit 22 and/or presents the bioinformatics on the display element 21, whereby the user can read the bioinformatics measured by the measurement device 10. For example, the host 20 may be a computer (such as a desktop computer or a notebook computer), a mobile internet device (such as a smart phone or a tablet computer), or a medical instrument dedicated or not dedicated to the measurement device 10.

In one embodiment, the host 20 may be selectively switched to a pairing mode or a beacon mode according to the number of the links to the measurement devices 10 to establish wireless links with the measurement devices 10. For example, while a single wireless link is established between the host 20 and a single measurement device 10, the host 20 is linked to the measurement device 10 in a pairing mode. The pairing mode may be realized with an existing communication protocol. For example, the measurement device 10 may be wirelessly linked to the host 20 with the Bluetooth communication protocol, the Zigbee communication protocol, the Thread communication protocol, or an IEEE 802.11ah communication protocol (e.g. the Sub-lGHz communication protocol).

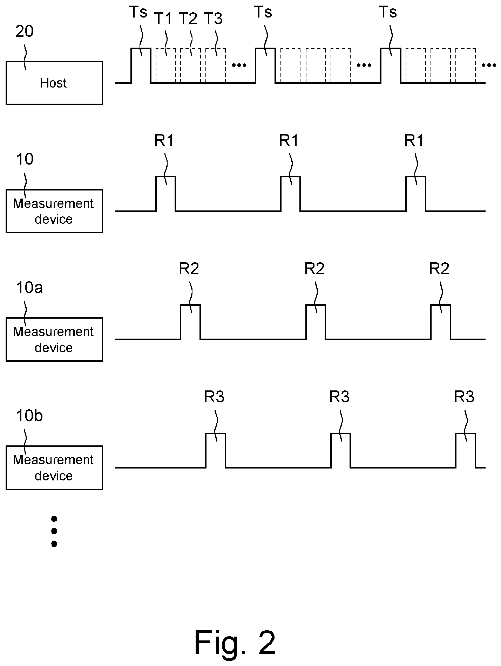

While a plurality of measurement devices 10, 10a and 10b are to be wirelessly linked to the host 20, the host 20 is switched to the beacon mode to communicate with the measurement devices 10, 10a and 10b. Refer to Fig. 2. In one embodiment, after the host 20 periodically sends out time synchronous signals Ts, the host 20 waits to receive the bioinformatics transmitted by the plurality of the measurement devices 10, 10a and 10b in sequence. For example, the host 20 may partition the interval between each two time synchronous signals Ts to have a plurality of transmission intervals T1, T2 and T3 and write the number of the transmission intervals into the time synchronous signals Ts. After the measurement devices 10, 10a and 10b receive the time synchronous signal Ts, the measurement devices 10, 10a and 10b respectively select the corresponding transmission intervals T1, T2 and T3 according to the time synchronous signal Ts to transmit bioinformatics. As shown in Fig. 2, the measurement device 10 transmits the bioinformatics R1 during the transmission interval T1; the measurement device 10a transmits the bioinformatics R2 during the transmission interval T2; the measurement device 10b transmits the bioinformatics R3 during the transmission interval T3. It is easily understood: the number of the transmission intervals T1, T2 and T3 should be equal to or greater than the number of the links between the host 20 and the measurement devices 10, 10a and 10b lest communication collision occur. In other words, different measurement devices 10, 10a and 10b respectively transmit bioinformatics during different transmission intervals T1, T2 and T3.

In one embodiment, the measurement devices 10, 10a and 10b may select the corresponding the transmission intervals T1, T2 and T3 according to their own device addresses. For example, the device address is divided by the number of the transmission intervals to acquire the remainder; the measurement device 10, 10a or 10b uses the remainder to select a transmission interval for transmitting bioinformatics. If different measurement devices 10, 10a and 10b select an identical transmission interval for communication, communication collision would occur. In such a case, one of the measurement devices 10, 10a and 10b may add "1" to its own remainder to avoid communication collision.

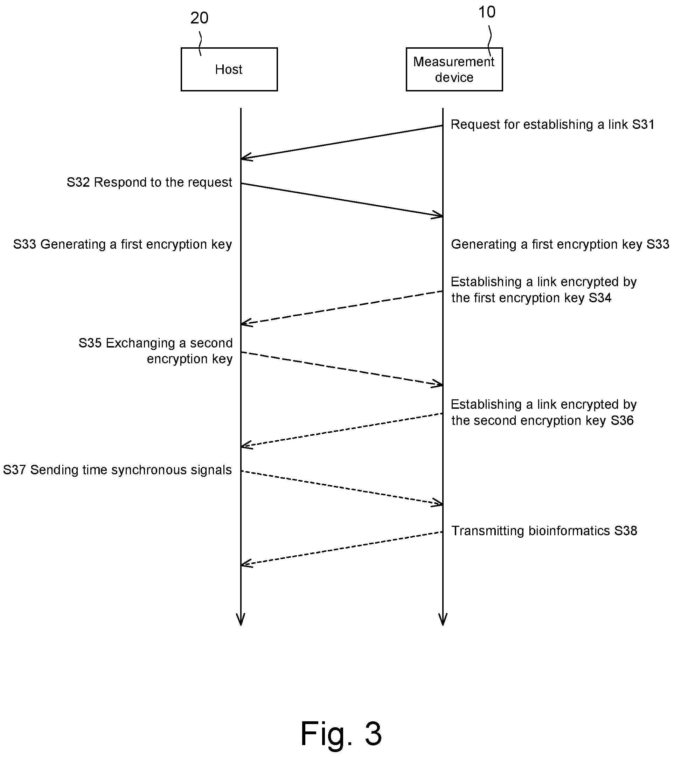

In one embodiment, for safety of data transmission, the communication link between the measurement device 10 and the host 20 may be an encrypted communication link, and the encryption method is applicable to the pairing mode and the beacon mode. Refer to Fig. 3 for the steps of establishing an encrypted wireless communication link between the measurement device 10 and the host 20. Firstly, the measurement device 10 sends a request to the host 20 for establishing an encrypted wireless communication link (Step S31). Next, the host 20 responds to the linking request of the measurement device 10 (Step S32). Next, the measurement device 10 and the host 20 respectively generate first encryption keys (Step S33). For example, the measurement device 10 and the host 20 may use the device address of the measurement device 10 to generate the first encryption keys. However, the present invention is not limited by the example. The measurement device 10 and the host 20 may also use the device address of the host 20 or the device addresses of the host 20 and the measurement device 10 to generate the first encryption keys. After the first encryption keys have been generated, the measurement device 10 and the host 20 can use the first encryption keys to encrypt and decrypt the data transmitted via the wireless communication link (Step S34). In a preferred embodiment, the measurement device 10 and the host 20 may exchange a second encryption key under the wireless communication link encrypted by the first encryption keys (Step S35). For example, the host 20 may generate the second encryption key to the measurement device 10; alternatively, the measurement device 10 may generate the second encryption key to the host 20. Next, the measurement device 10 and the host 20 may use the second encryption key to encrypt and decrypt the data transmitted via the wireless communication link (Step S36). For example, the second encryption key is used while the host 20 sends out the time synchronous signal (Step S37) and while the measurement device 10 transmits bioinformatics (Step S38). In one embodiment, the host 20 and the measurement device 10 may periodically or non-periodically demand the measurement device 10 to update the second encryption key so as to enhance the safety of data transmission.

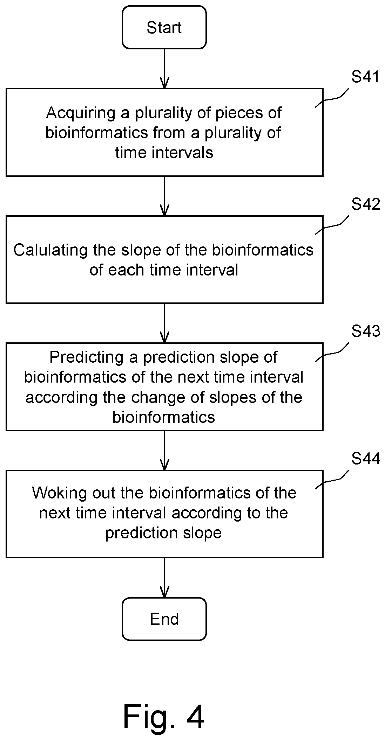

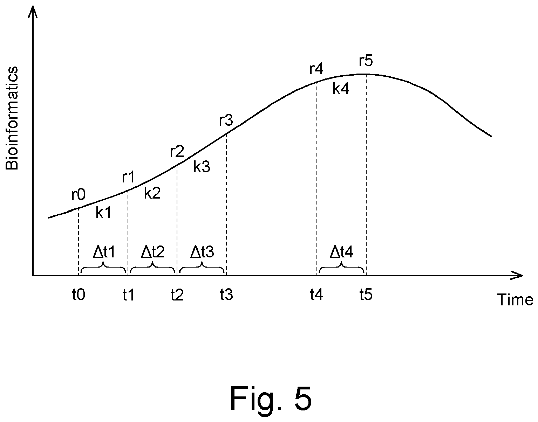

While the measurement device 10 is used to measure the blood glucose and/or blood ketone of the sweat or tissue fluid, a lag phenomenon may occur. In order to make the measured bioinformatics approach the current bioinformatics, the bioinformatics measured by the measurement device 10 should be calibrated. Refer to Fig. 4 for illustrating a bioinformatics calibration method according to one embodiment of the present invention. Firstly, an electronic device is used to acquire a plurality of pieces of bioinformatics, such as lagging bioinformatics, of an individual subject respectively in a preceding time interval and a current time interval (Step S41). Next, the measurement device calculates the slope of the bioinformatics of each time interval (Step S42). Refer to Fig. 5 also. In an example, the measurement device respectively acquires three pieces of bioinformatics r0, r1 and r2 at three time points t0, t1 and t2 and then works the bioinformatics slopes k1 and k2 respectively corresponding to the time intervals Δt1 and Δt2.

Next, the measurement device predicts a prediction slope of bioinformatics of the next time interval according to the change of bioinformatics slopes (Step S43). For example, while the absolute value of the slope (such as k2) of the current time interval (such as Δt2) is greater than the absolute value of the slope (such as k1) of the preceding time interval (such as Δt1), it indicates that the value of bioinformatics rises gradually and then dramatically, as shown in Fig. 5, or descends gradually and then dramatically. In such a case, the prediction slope k3 may be obtained via multiplying the slope (k2) of the current time interval (Δt2) with a preset value. Contrarily, while the absolute value of the slope of the current time interval is smaller than the absolute value of the slope of the preceding time interval, it indicates that the value of bioinformatics rises dramatically and then gradually or descends dramatically and then gradually. In such a case, the prediction slope may be obtained via dividing the slope of the current time interval with a preset value. While the absolute value of the slope of the current time interval is equal to the absolute value of the slope of the preceding time interval, the prediction slope of the next time interval is equal to the slope of the current interval. It is easily understood: the preset value may be modified according to the length of the time interval, the measured bioinformatics, or other background factors. The preset value may also be dynamically modified according to the ratios of the slopes of a plurality of time intervals. Then, the measurement device works out the bioinformatics of the next time interval according to the prediction slope (Step S44). For example, suppose that the prediction slope of the next time interval (such as Δt3) is k3 and that the bioinformatics measured at the time point t2 is r2. According to the bioinformatics r2 measured at the time point t2 and the prediction slope k3, the bioinformatics r3 of the time point t3, which is behind the next time interval Δt3, may be predicted.

While the absolute value of the slope of the current time interval is smaller than a threshold, it may be determined that the trend of the measured bioinformatics will be reversed. For example, the trend will change from rising to descending, or the trend will change from descending to rising. Therefore, the prediction slope may be the negative value of the slope of the current time interval. Refer to Fig. 5 again. In an example, the measurement device acquires the bioinformatics r4 and r5 at the time points t4 and t5 and works out the slope k4 of the time interval Δt4. Because the absolute value | k4 | of the slope k4 is smaller than a threshold, the prediction slope of the next time interval may be the negative value of the slope k4, i.e. the trend is reversed.

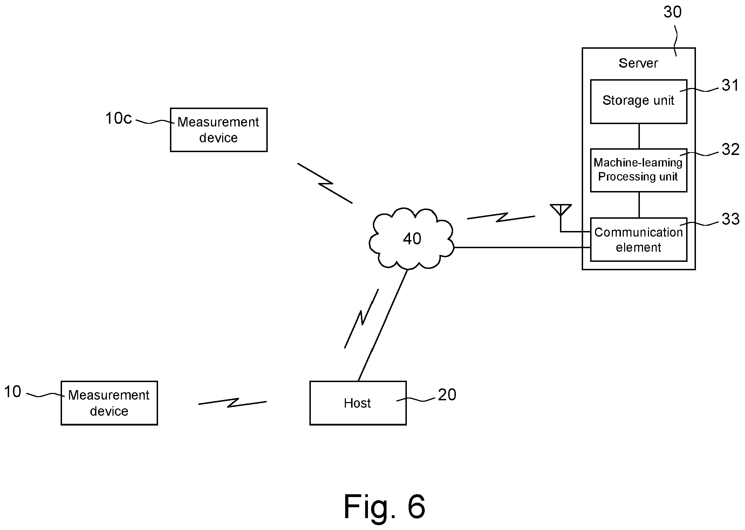

The bioinformatics calibration method of the present invention does not need a high computational ability (such as matrix operation). Therefore, a measurement device only having a limited computational ability and a limited memory size is also sufficient to realize the bioinformatics calibration method of the present invention. However, the present invention is not limited by the abovementioned example. Refer to Fig. 6. In one embodiment, the bioinformatics measurement system of the present invention comprises at least one measurement device 10, a host 20 and a cloud server 30. The host 20 and the server 30 may also realize the bioinformatics calibration method of the present invention. In an example, the measurement device 10 transmits bioinformatics to the host 20, whereby the host 20 can realize the bioinformatics calibration method of the present invention. In another example, the host 20 is linked to the server 30 through a mobile communication network or the Internet 40 by a wired network interface or a wireless network interface and transmits the bioinformatics measured by the measurement device 10 to the server 30, whereby the server 30 can realize the bioinformatics calibration method of the present invention. Then, the server 30 transmits the prediction results back to the host 20, and the user can read the prediction results.

In the abovementioned embodiment, the measurement device 10 transmits the measured bioinformatics to the server 30 through the host 20. However, the present invention is not limited by the abovementioned embodiment. Refer to Fig. 6 again. In one embodiment, a network communication link can be can be directly established between the measurement device 10c and the server 30 through a Low Power Wide Area Network (LPWAN) for transmitting the bioinformatics measured by the measurement device 10c. The Low Power Wide Area Network may be LoRa, Sigfox, or NB-IoT, which features small transmitted data volume, long transmission distance, and low power consumption. Therefore, LPWAN is suitable for the measurement device of the present invention.

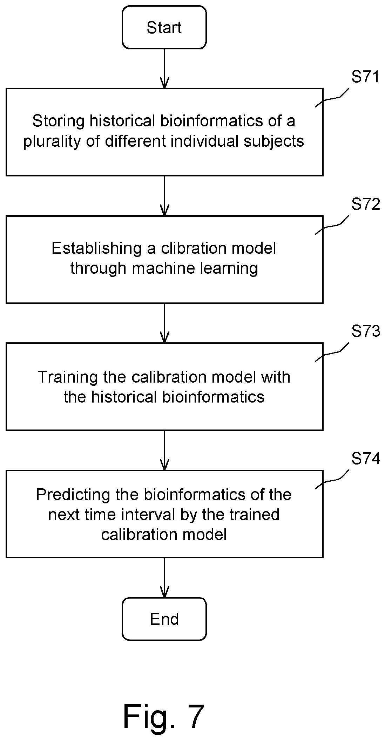

In one embodiment, the server 30 may further perform a prediction calibration on the bioinformatics measured by the measurement device. Refer to Fig. 6 and Fig. 7. In one embodiment, the server 30 includes a storage unit 31, a machine-learning processing unit 32 and a communication element 33. The communication element 33 is a wired or wireless network interface linked to the Internet 40 for receiving the bioinformatics measured by the measurement device 10c. Firstly, the server 30 stores the received bioinformatics of a plurality of different individual subjects in the storage unit 31 to function as the historical bioinformatics (Step S71). Next, the machine-learning processing unit 32 establishes a calibration model through machine learning (Step S72). For example, the calibration model is established based on a Recurrent Neural Network (RNN). Next, the server 30 uses the stored historical bioinformatics of the plurality of different individual subjects to train the calibration model (Step S73). Next, the server 30 uses the trained calibration model and a plurality of pieces of bioinformatics of a specified individual subject to predict the bioinformatics of the specified individual subject in the next time interval (Step S74). Then, the server 30 transmits the predicted bioinformatics to the host 20 where the user may read the bioinformatics.

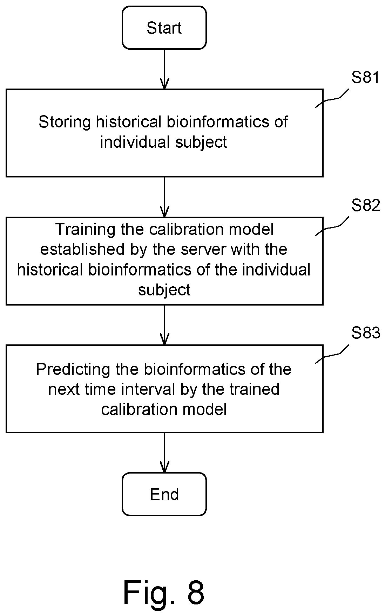

In one embodiment, the server 30 may provide the calibration model for the host 20, whereby the host 20 can further perform calibration and prediction on the measured bioinformatics. Refer to Fig. 8 for an example of this embodiment. Firstly, the host 20 stores a plurality of pieces of bioinformatics of a single subject as the individual subject historical bioinformatics of the single subject (Step S81). Next, the host 20 uses the individual subject historical bioinformatics of the single subject to train the calibration model established by the server 30 (Step S82). Next, the host 20 uses the trained calibration model and a plurality of pieces of bioinformatics of the specified subject to predict the bioinformatics of the specified subject in the next time interval (Step S83). Then, the host 20 presents the bioinformatics on the display element.

In conclusion, the bioinformatics measurement system of the present invention may selectively switch to a pairing mode or a beacon mode according to the number of the links with the measurement devices. Therefore, the bioinformatics measurement system of the present invention is adapted to different application scenarios. Further, the bioinformatics calibration method of the present invention may predict the bioinformatics of the next time interval according to the slope change of bioinformatics to make the predicted bioinformatics approach the current bioinformatics. Furthermore, the bioinformatics calibration method of the present invention may be realized with measurement devices with lower computation ability.

While the invention is susceptible to various modifications and alternative forms, a specific example thereof has been shown in the drawings and is herein described in detail. It should be understood, however, that the invention is not to be limited to the particular form disclosed, but to the contrary, the invention is to cover all modifications, equivalents, and alternatives falling within the appended claims.

## Claims

1. A bioinformatics measurement system **characterized in** comprising:
at least one measurement device used to measure bioinformatics of an individual subject; and
a host receiving bioinformatics measured by the at least one measurement device through at least one wireless communication link established between the host and the at least one measurement device, wherein the host is selectively switched to a pairing mode or a beacon mode to establish the at least one wireless communication link with the at least one measurement devices according to a number of links between the host and the at least one measurement device.

2. The bioinformatics measurement system according to claim 1 **characterized in that** the wireless communication link follows a Bluetooth communication protocol, a Zigbee communication protocol, a Thread communication protocol, or an IEEE 802.11ah communication protocol

3. The bioinformatics measurement system according to claim 1 **characterized in that** the host sends out a time synchronous signal periodically and then waits to receive the bioinformatics sent back by the plurality of measurement devices in sequence while wireless communication links are established between the host and the plurality of measurement devices in the beacon mode.

4. The bioinformatics measurement system according to claim 3 **characterized in that** the host partitions a time interval between the time synchronous signals to have a plurality of transmission intervals and writes a count of the transmission intervals into the time synchronous signals.

5. The bioinformatics measurement system according to claim 4 **characterized in that** the number of the transmission intervals is equal to or greater than the number of the links between the host and the measurement devices, and each of the measurement devices selects the corresponding transmission interval to send back the bioinformatics.

6. The bioinformatics measurement system according to claim 1 **characterized in that** the host is computer, a mobile internet device, or a medical instrument.

7. The bioinformatics measurement system according to claim 1 **characterized in that** the bioinformatics is at least one of blood glucose and blood ketone.

8. The bioinformatics measurement system according to claim 1 **characterized in that** the host and the at least one measurement device generate a first encryption key according to at least one of device addresses of the host and the measurement device, and the first encryption key is used to encrypt the wireless communication link.

9. The bioinformatics measurement system according to claim 8 **characterized in that** the host and the measurement device exchange a second encryption key through the wireless communication link encrypted by the first encryption key and then encrypt the wireless communication link by the second encryption key for transmitting the bioinformatics, wherein the second encryption key is updated periodically or non-periodically.

10. A bioinformatics calibration method **characterized in** comprising steps:
acquiring a plurality of pieces of bioinformatics of an individual subject respectively in a preceding time interval and a current time interval by an electronic device, wherein the bioinformatics is lagging bioinformatics;
working out slopes of bioinformatics of the preceding time interval and the current time interval by the electronic device according to the plurality of pieces of bioinformatics;
predicting a prediction slope of bioinformatics of a next time interval by the electronic device according to a change of the slopes of the preceding time interval and the current time interval; and
working out bioinformatics behind the next time interval by the electronic device according to the prediction slope.

11. The bioinformatics calibration method according to claim 10 **characterized in that** while the slope of the current time interval is equal to the slope of the preceding time interval, the prediction slope is the slope of the current time interval.

12. The bioinformatics calibration method according to claim 10 **characterized in that** while an absolute of the slope of the current time interval is greater than an absolute value of the slope of the preceding time interval, the prediction slope is generated via multiplying the slope of the current time interval with a preset value.

13. The bioinformatics calibration method according to claim 10 **characterized in that** while an absolute of the slope of the current time interval is smaller than an absolute value of the slope of the preceding time interval, the prediction slope is generated via dividing the slope of the current time interval with a preset value.

14. The bioinformatics calibration method according to claim 10 **characterized in that** while an absolute of the slope of the current time interval is smaller than a threshold, the prediction slope is a negative value of the slope of the current time interval.

15. The bioinformatics calibration method according to claim 10 **characterized in** further comprising steps:
storing a plurality of pieces of bioinformatics of a plurality of different individual subjects by a server to function as historical bioinformatics;
establishing a calibration model through machine learning by the server;
training the calibration model with the historical bioinformatics by the server; and
predicting bioinformatics of the individual subjects in the next time interval by the server based on the trained calibration model and the plurality of pieces of bioinformatics of the individual subjects.

16. The bioinformatics calibration method according to claim 15 **characterized in that** the calibration model is established based on a Recurrent Neural Network (RNN).

17. The bioinformatics calibration method according to claim 15 **characterized in that** the electronic device is a measurement device and that a network communication link is established between the measurement device and the server through a Low Power Wide Area Network (LPWAN) for transmitting the plurality of pieces of bioinformatics of the individual subjects, which are measured by the measurement device.

18. The bioinformatics calibration method according to claim 15 **characterized in** further comprising steps:
storing the plurality of pieces of bioinformatics of the individual subject by a host to function as individual subject historical bioinformatics;
training the calibration model with the individual subject historical bioinformatics by the host; and
predicting bioinformatics of the individual subject in the next time interval by the host based on the trained calibration model and the plurality of pieces of bioinformatics of the individual subject.

19. The bioinformatics calibration method according to claim 18 **characterized in that** the electronic device is a measurement device and that the measurement device is wirelessly linked to the host with a Bluetooth communication protocol, a Zigbee communication protocol, a Thread communication protocol, or an IEEE 802.11ah communication protocol for transmitting the plurality of pieces of bioinformatics of the individual subject, which are measured by the measurement device.

20. The bioinformatics calibration method according to claim 10 **characterized in that** the electronic device is a measurement device and that the measurement device includes a measurement element used to measure the plurality of pieces of bioinformatics of the individual subject.
